# EUROPÄISCHE PATENTSCHRIFT

(11) **EP 1 997 968 B1**
(45) Veröffentlichungstag und Bekanntmachung des Hinweises auf die Patenterteilung: **02.01.2019**
(21) Anmeldenummer: 08008800.8
(22) Anmeldetag: 10.05.2008
(51) Int. Cl.: E03C 1/084, F16K 11/00

(54) **Eine Niederdruck-Sanitäreinheit zur Verwendung mit einem drucklosen Warmwasserspeicher und einem Stahlregler**
A low pressure sanitary unit for use with a pressureless hot water boiler and steel controller
Une unité sanitaire base pression destinée à être utilisée avec un accumulateur d'eau chaude sans pression et un régulateur en acier

(30) Priorität: 01.06.2007 DE 102007025725
(43) Veröffentlichungstag der Anmeldung: 03.12.2008
(73) Patentinhaber: Neoperl GmbH, 79379 Müllheim (DE)
(72) Erfinder: Schmidt, Heinz, 70567 Stuttgart (DE)
(74) Vertreter: Maucher Jenkins Patent- und Rechtsanwälte

(56) Entgegenhaltungen:
- EP-A1- 1 577 259
- DE-A1- 3 338 063
- DE-C1- 4 222 978
- DE-U1- 7 828 157
- DE-U1- 29 502 162

## Beschreibung

Die Erfindung betrifft eine Niederdruck-Sanitärarmatur zur Verwendung mit einem drucklosen Warmwasserspeicher mit
a) einem Wasserauslauf und
b) einem Strahlregler, mit
   - einem Strahlreglergehäuse;
   - einer Einrichtung, welche das Nachtropfen von Wasser bei geschlossener Sanitärarmatur verhindert.

Derartige marktbekannte Strahlregler werden dort eingesetzt, wo das Wasser nahezu drucklos durch einen Wasserauslauf geführt wird. Insbesondere bei drucklosen Warmwasserspeichern soll der zulässige Staudruck im Wasserauslauf 0,8 bar nicht überschreiten. Die derzeit hierfür verwendeten Strahlregler sind daher mit einem Druckausgleichsmittel in Form von genügend großen Durchtrittslöchern für das Wasser ausgestattet, das einen Staudruck im Wasserauslauf und damit in dem Warmwasserspeicher verhindert. Das Druckausgleichsmittel läßt ferner einen Luftaustausch mit der Umgebung zu, um einen Unterdruck im Wasserauslauf auszugleichen, welcher insbesondere beim Abkühlen des Warmwasserspeichers entsteht. Bei solchen Strahlreglern tropft jedoch nach einer Wasserentnahme die Sanitärarmatur bis zu einigen Minuten nach, bis sich der Wasserauslauf entleert hat.

Die DE 33 38 063 A1 beschreibt eine Niederdruck-Sanitärarmatur ohne einen Strahlregler.

Ferner beschreibt die DE 78 28 157 U ein Dosierventil für Flüssigkeiten, das eine Lochplatte aufweist. Die Lochplatte ist mit einer Vielzahl von Bohrungen kleinen Querschnitts ausgestattet, um ein Nachtropfen zu verhindern.

Um das Nachtropfen zu verhindern, weisen andere bekannte Strahlregler Rückflussverhinderer-Ventile auf, die jedoch einen großen Einbauraum erfordern und so insbesondere für flache Wasserausläufe nicht geeignet sind.

Aufgabe der vorliegenden Erfindung ist es, einen Strahlregler für eine Niederdruck-Sanitärarmatur der eingangs genannten Art so auszugestalten, dass mit einfachen und kompakten Bauteilen ein Überdruck und ein Unterdruck im Wasserauslauf und gegebenenfalls im Warmwasserspeicher ausgeschlossen wird und ein Nachtropfen verhindert wird.

Diese Aufgabe wird erfindungsgemäß dadurch gelöst, dass die Einrichtung zum Verhindern des Nachtropfens eine Strahlscheibe mit einer Vielzahl von Durchtrittslöchern umfasst, die in und entgegen der Fließrichtung des Wassers relativ zu dem Strahlreglergehäuse bewegbar angeordnet ist, wobei ein vorgespanntes elastisches Element die Strahlscheibe mit einer Dichtungsfläche entgegen der Fließrichtung unterhalb eines vorgegebenen maximalen Fließdrucks gegen eine gehäusefeste Dichtungsfläche presst und die Durchtrittslöcher in Größe und/oder Verteilung derart angeordnet sind, dass die Strahlscheibe bei geschlossener Sanitärarmatur den Strahlregler tropfdicht abschließt, bei einer Wasserentnahme das Wasser durch die Durchtrittslöcher strömt und bei Erreichen des vorgegebenen maximalen Fließdrucks die Dichtungsfläche der Strahlscheibe entgegen der Vorspannung von der gehäusefesten Dichtungsfläche abhebt, so dass das Wasser zwischen den Dichtungsflächen durchströmen kann.

Erfindungsgemäß ist also eine flachbauende und kompakte Strahlscheibe vorgesehen, deren Durchtrittslöcher in Größe und Verteilung derart angeordnet sind, dass bei einer Wasserentnahme unterhalb des vorgegebenen maximalen Fließdrucks das Wasser die Durchtrittslöcher druckfrei durchströmt. Das elastische Element preßt dabei die Strahlscheibe gegen den Fließdruck des Wassers gegen die gehäusefeste Dichtungsfläche. Der maximale Fließdruck ist durch die Vorspannung des elastischen Elements vorgegeben. Wird der Wässerfluss gestoppt, so hält die Strahlscheibe wegen der besonderen Anordnung der Durchtrittslöcher die Wassersäule tropfdicht im Wasserauslauf, wodurch ein Nachtropfen verhindert wird. Kühlt das Wasser im Wasserauslauf und gegebenenfalls im Warmwasserspeicher ab, so wird ein dadurch entstehender Unterdruck über die Durchtrittslöcher gegenüber der Umgebung ausgeglichen. Liegt an der Strahlscheibe ein den vorgegebenen maximalen Fließdruck übersteigender Fließdruck an, so hebt sich die Strahlscheibe gegen die Spannkraft des elastischen Elements von der gehäusefesten Dichtungsfläche ab. Das Wasser strömt dann seitlich zwischen den Dichtungsflächen durch und gleicht so den Überdruck im Wasserauslauf und gegebenenfalls im Warmwasserspeicher aus. Eine Vergrößerung des Fließdrucks kann durch entsprechende Erhöhung des Wasserflusses, aber auch durch Verschmutzung, insbesondere Verkalkung, der Strahlscheibe verursacht werden. Insgesamt wirkt also die mit den Durchtrittslöchern speziell ausgestaltete Strahlscheibe zusammen mit dem elastischen Element als Einrichtung, welche sowohl unerwünschte Überdrücke und Unterdrücke im Wasserauslauf und damit im Warmwasserspeicher als auch ein Nachtropfen des Wassers verhindert.

Bei einer vorteilhaften Ausführungsform gemäß Anspruch 2 kann das elastische Element einfach eine Druckfeder sein, die mit einem Ende insbesondere im Zentrum der Strahlscheibe angreift und mit dem anderen Ende an einem gehäusefesten Teil anliegt. Eine Druckfeder ist robust, verschleißarm und störungsunanfällig.

Bei einer weiteren vorteilhaften Ausführungsform gemäß Anspruch 3 kann die Druckfeder auf einem zum Strahlreglergehäuse koaxialen Führungsbolzen sitzen, der mit der Strahlscheibe relativ zu dem Strahlreglergehäuse bewegbar ist. Der Führungsbolzen dient der Führung des elastischen Elements, insbesondere der Druckfeder. Außerdem kann über die Position des Führungsbolzens im Strahlreglergehäuse ein Überschreiten des vorgegeben Fließdrucks einfach erkannt werden. Der Führungsbolzen kann so auch Teil einer Verschmutzungsanzeige sein, die einen Hinweis darauf gibt, wenn die Durchtrittslöcher der Strahlscheibe durch Schmutz oder Kalk verstopft sind.

Zweckmäßigerweise kann hierzu gemäß Anspruch 4 der Strahlregler ein Anzeigemittel aufweisen, mit dem die Position des Führungsbolzens einfach nach außen sichtbar gemacht werden.

Vorteilhafterweise kann gemäß Anspruch 5 die gehäusefeste Dichtungsfläche von einem Dichtungsring mit gebildet sein, der an dem Strahlreglergehäuse auf der Wasserauslauf zugewandten Stirnseite ohnehin angeordnet ist. So kann auf eine separate Dichtungsfläche verzichtet werden.

Ferner kann gemäß Anspruch 6 die Strahlscheibe bei Überschreiten eines Fließdrucks von 0,5 bar abheben.

Um ein Nachtropfen optimal zu verhindern, können gemäß Anspruch 7 die Durchtrittslöcher wabenartig angeordnet sein.

Weitere Vorteile, Merkmale und Einzelheiten der Erfindung ergeben sich aus der nachfolgenden Beschreibung, in der ein Ausführungsbeispiel der Erfindung anhand der Zeichnung näher erläutert wird; es zeigen
- Figur 1: schematisch eine isometrische Darstellung eines Strahlreglers für eine Niederdruck-Sanitärarmatur mit einer federvorgespannten Strahlscheibe;
- Figur 2: schematisch eine Seitenansicht des Strahlreglers aus Figur 1;
- Figur 3: einen Axialschnitt des Strahlreglers aus Figur 2 entlang der dortigen Linie III-III;
- Figur 4: eine isometrische Darstellung eines Strahlreglereinsatzes des Strahlreglers aus den Figuren 1 bis 3;
- Figur 5: eine Detailansicht des Strahlreglereinsatzes aus Figur 4 im dortigen Bereich V.

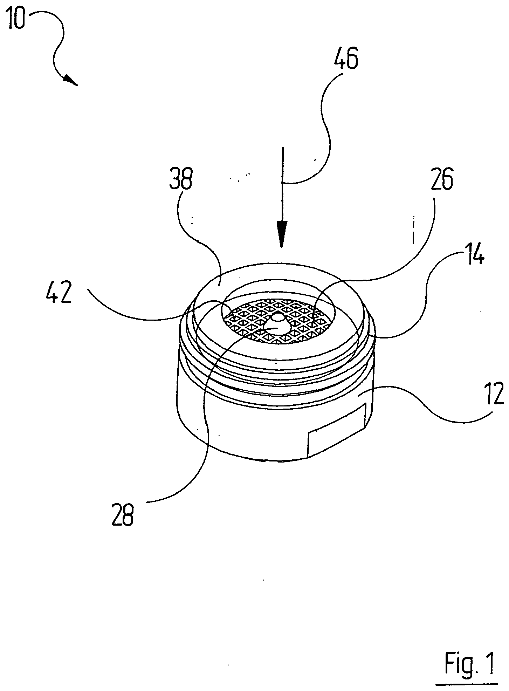

In Figur 1 ist ein insgesamt mit dem Bezugszeichen 10 versehener Strahlregler dargestellt, welcher am Ausgang eines nicht dargestellten Wasserauslaufs einer Niederdruck-Sanitärarmatur, die einem ebenfalls nicht gezeigten drucklosen Warmwasserspeicher zugeordnet ist.

Der Strahlregler 10 umfasst ein Strahlreglergehäuse 12 in Form eines hohlen Kreiszylinders, der an beiden Stirnseite offen ist. Die äußere Umfangsseite des Strahlreglergehäuse 12 weist im Bereich ihrer dem Wasserauslauf zugewandten Stirnseite, in Figur 1 oben, ein Außengewinde 14 auf, das in ein entsprechendes Innengewinde im Wasserauslauf eingeschraubt werden kann.

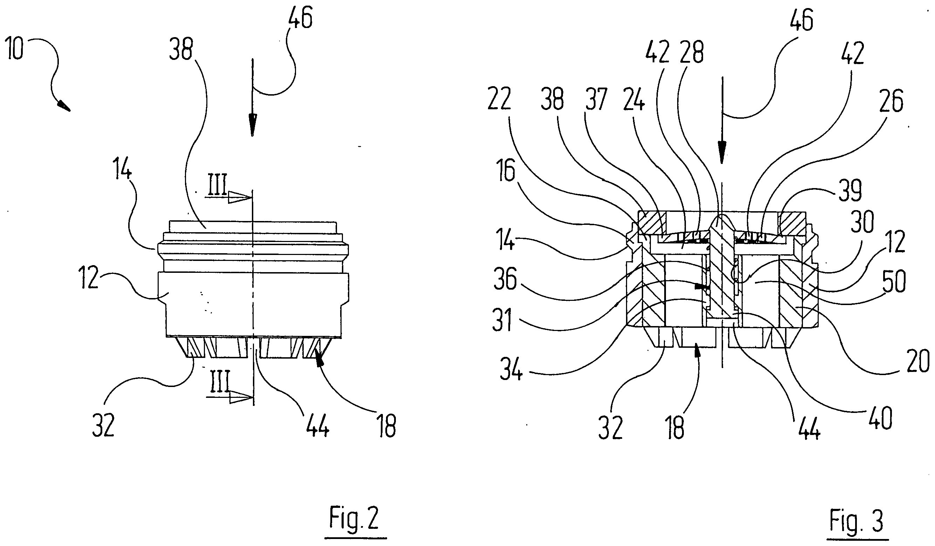

Das Strahlreglergehäuses 12 weist im Bereich seiner dem Wasserauslauf zugewandten Stirnseite an der Innenseite einen umlaufenden Absatz 16 auf, der im Axialschnitt in Figur 3 oben gezeigt ist.

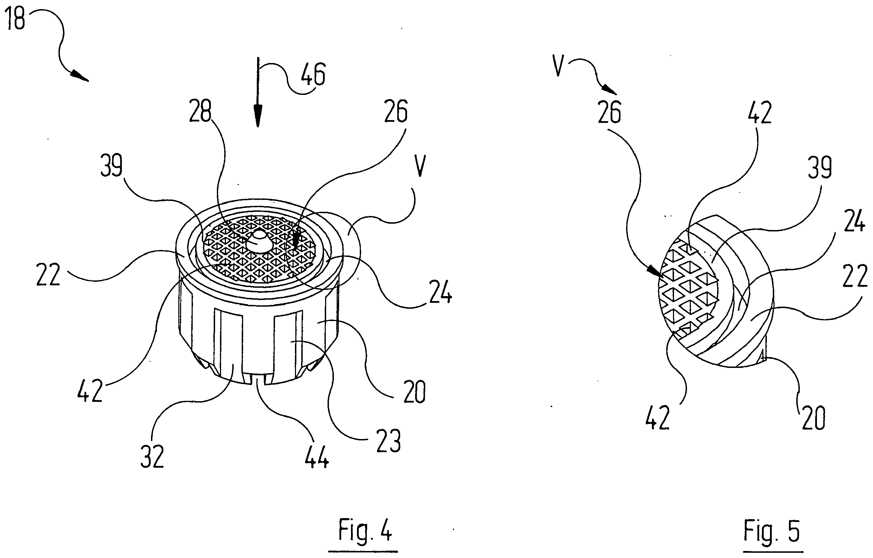

Von der dem Wasserauslauf zugewandten Stirnseite aus ist ein insbesondere in Figur 4 gezeigter Strahlreglereinsatz 18 koaxial in das Strahlreglergehäuse 12 eingesetzt, dessen Einsatzgehäuse 20 ebenfalls etwa die Form eines hohlen Kreiszylinders hat.

Der Außendurchmesser des Einsatzgehäuses 20 entspricht dem Innendurchmesser des Strahlreglergehäuse 12, so dass die angrenzenden Mantelflächen aneinander anliegen.

Das Einsatzgehäuse 20 weist an seiner dem Wasserauslauf zugewandten Stirnseite einen zu dem Absatz 16 komplementären Kragen 22 auf, der an dem Absatz 16 des Strahlreglergehäuses 12 anliegt und ein Durchrutschen des Strahlreglereinsatzes 18 verhindert.

Die Außenmantelfläche des Einsatzgehäuses 20 (Figur 4) weist eine Vielzahl von quaderförmigen Rücksprüngen 23 auf, welche auf der Innenmantelfläche des Einsatzgehäuses 20 erhaben sind und strahlformend wirken. Die Rücksprünge 23 erstrecken sich in axialer Richtung auf der dem Wasserauslauf abgewandten Seite des Kragens 22 bis zur dortigen Stirnseite des Einsatzgehäuses 20 und gehen dort in keilförmige, nach unten über das Strahlreglergehäuse 12 überstehende Nasen 32 über.

Der dem Wasserauslauf zugewandte Innenbereich des Einsatzgehäuses 20 ist gegenüber dem restlichen Innenbereich querschnittserweitert und begrenzt eine Kammer 24 mit, in der eine in der Draufsicht kreisrunde Strahlscheibe 26 angeordnet ist. Der Durchmesser der Strahlscheibe 26 ist kleiner als der Durchmesser der Kammer 24 und größer als der Durchmesser des restlichen Innenbereichs des Einsatzgehäuses 20.

Die Strahlscheibe 26 sitzt fest auf einem Federführungsbolzen 28, welcher koaxial zum Strahlreglergehäuse 12 und zum Einsatzgehäuse 20 in letzterem angeordnet ist.

Der Federführungsbolzen 28 führt mit einem kegelig geformten Ende durch eine entsprechende mittige Öffnung der Strahlscheibe 26.

Der Federführungsbolzen 28 ist auf der der Strahlscheibe 26 abgewandten Seite in einer Stufenbohrung 30 eines zylindrischen Mittelbereichs 31 geführt. Der Mittelbereich ist koaxial zum Einsatzgehäuse 20 mit seiner dem Wasserauslauf abgewandten unteren Stirnseite, in Figur 3 unten, mit den keilförmigen Nasen 32 verbunden.

Die Innenfläche des Stufenbohrung 30 weist in ihrer dem Wasserauslauf abgewandten axialen Hälfte einen umlaufenden Auflagekragen 34 auf. Auf der dem Wasserauslauf zugewandten Oberseite des Auflagekragens 34 liegt eine vorgespannte Druckfeder 36 an. Die Druckfeder 36 sitzt koaxial auf dem Federführungsbolzen 28 und wird durch diesen geführt.

Das freie, dem Wasserauslauf zugewandte Ende der Druckfeder 36 greift von der dem Wasserauslauf abgewandten Seite an die Strahlscheibe 26 an und drückt diese in Richtung zum Wasserauslauf.

Die Druckfeder 36 ist so ausgestaltet, dass sie ab einem Fließdruck von etwa 0,5 bar von der Seite des Wasserauslaufs auf die Strahlscheibe 26 nachgibt.

An der dem Wasserauslauf zugewandten Fläche des Kragens 22 liegt flächig ein Dichtungsring 38 an, welcher den Strahlregler 10 gegenüber dem Wasserauslauf abdichtet.

Der Außendurchmesser des Dichtungsrings 38 entspricht dem Außendurchmesser des Kragens 22. Der Innendurchmesser des Dichtungsrings 38 ist kleiner als der Innendurchmesser der Kammer 24 und der Außendurchmesser der Strahlscheibe 26.

An der dem Wasserauslauf abgewandten Seite des Dichtungsrings 38 liegt die dem Wasserauslauf zugewandte Oberseite der Strahlscheibe 26 an; diese wird durch die Vorspannung der Druckfeder 36 dicht gegen den Dichtungsring 38 gepresst. Die einander überlappenden Oberflächenbereiche der Strahlscheibe 26 und des Dichtrings 38 wirken dabei als Dichtungsflächen 37 beziehungsweise 39.

Das dem Wasserauslauf abgewandten Ende des Federführungsbolzens 28 befindet auf der dem Wasserauslauf abgewandten Seite des Auflagekragens 34 und ist als Bolzenkopf 40 ausgebildet. Der Außendurchmesser des Bolzenkopfs 40 ist größer als der Innendurchmesser des Auflagekragens 34, so dass der Bolzenkopf 40 verhindert, dass der Federführungsbolzen 28 bei entferntem Dichtring 38 in Richtung auf den Wasserauslauf zu aus der Stufenbohrung 30 rutscht.

Die Strahlscheibe 26 weist eine Vielzahl von in den Figuren 1, 4 und 5 gezeigten Wasserdurchtrittslöchern 42 auf, welche in besonderer Weise wabenartig angeordnet sind, derart, dass Wasser unter einem Fließdruck von maximal etwa 0,5 bar die Strahlscheibe 26 durchströmen kann. Wenn das Wasser bei gestoppter Wasserzufuhr lediglich-mit dem statischem Druck der Wassersäule im Wasserauslauf beaufschlagt ist, ist die Strahlscheibe 26 aufgrund von Oberflächenspannungseffekten für Wasser dicht. Luft kann hingegen in beiden Richtungen durch die Wasserdurchtrittslöcher 42 strömen.

Die Stufenbohrung 30 ist außerdem auf ihrer dem Wasserauslauf abgewandten Seite offen, so dass die Position des Bolzenkopfs 40 und damit des Federführungsbolzens 28 von außen sichtbar und so eine Verschmutzungsanzeige 44 realisiert ist.

Der Strahlregler 10 funktioniert wie folgt:
In druckfreiem Zustand, wenn das oder die Ventil(e) der Sanitärarmatur geschlossen sind, drückt die vorgespannte Druckfeder 36 die Strahlscheibe 26 dicht gegen den Dichtungsring 38.

Sobald der Wasserfluss frei gegeben wird, strömt das Wasser aus dem Wasserauslauf in Richtung des Pfeils 46 durch die Wasserdurchtrittslöcher 42 in der Strahlscheibe 26, fließt durch die Kammer 24 und einen Ringraum 50 zwischen dem Mittelbereich 31 und der Innenseite des Einsatzgehäuses 20 und durch die offene Unterseite des Einsatzgehäuses 20 aus dem Strahlreglereinsatz 18.

Wenn der Wasserfluss gestoppt wird, so wird wegen der speziellen Anordnung der Wasserdurchtrittslöcher 22 die oberhalb der Strahlscheibe 26 noch befindliche Wassersäule tropfdicht im Wasserauslauf gehalten, so dass ein Nachtropfen verhindert wird.

In Anschluß an eine Abnahme von warmem Wasser kühlen das im Wasserauslauf noch befindliche Wasser und der Warmwasserspeicher ab, was zu einem Unterdruck im Wasserauslauf führen könnte. Durch die Wasserdurchtrittslöcher 42 strömt jedoch Umgebungsluft dem Wasserauslauf zu, wodurch die Ausbildung eines nennenswerten Unterdrucks verhindert wird.

Wenn bei einer Wasserentnahme der durch die Druckfeder 38 und die Ausgestaltung der Strahlscheibe 26 vorgegebene maximale Fließdruck von 0,5 bar überschritten wird, beispielsweise wenn die Wasserdurchtrittslöcher 42 durch Schmutz und/oder Kalk ganz oder teilweise verschlossen sind oder die zulaufende Wassermenge zu groß ist, hebt die Strahlscheibe 26 gegen die Federvorspannung der Druckfeder 36 von dem Dichtungsring 38 ab. Das Wasser fließt nun zwischen der Dichtungsfläche 37 des Dichtungsrings 38 und der Dichtungsfläche 39 der Strahlscheibe 26 radial nach außen in die Kammer 24, von dort in den Ringraum 50 und verläßt durch die offene Unterseite des Einsatzgehäuses 20 den Strahlreglereinsatz 18. Die federbelastete Strahlscheibe 26 wirkt somit als Überdruckventil und verhindert einen Überdruck im Wasserauslauf und im Warmwasserspeicher.

Beim Abheben der Strahlscheibe 26 wird gleichzeitig der Federführungsbolzen 28 relativ zum Mittelbereich 31 axial in Fließrichtung 46 verschoben, so dass der Bolzenkopf 40 von außen sichtbar ist. Somit kann optisch erfasst werden, sobald der Fließdruck überschritten wird und gegebenenfalls auf eine Verschmutzung beziehungsweise Verkalkung der Strahlscheibe 26 geschlossen werden.

Bei dem oben beschriebenen Ausführungsbeispiel eines Strahlreglers sind unter anderem folgende Modifikationen möglich:
Anstelle der Druckfeder 36 kann auch ein andersartiges elastisches Element, beispielsweise ein Gummielement, verwendet werden.

Auf den Federführungsbolzen 28 und die Verschmutzungsanzeige 44 kann auch verzichtet werden.

Die Strahlscheibe 26 kann statt gegen den Dichtungsring 38 auch gegen eine entsprechende an dem Einsatzgehäuse 20 angeordnete Dichtungsfläche gepresst werden.

## Patentansprüche

1. Niederdruck-Sanitärarmatur zur Verwendung mit einem drucklosen Warmwasserspeicher mit
a) einem Wasserauslauf und
b) einem Strahlregler
- einem Strahlreglergehäuse (12);
- einer Einrichtung (26, 36), welche das Nachtropfen von Wasser bei geschlossener Sanitärarmatur verhindert,
**dadurch gekennzeichnet, dass**
die Einrichtung zum Verhindern des Nachtropfens eine Strahlscheibe (26) mit einer Vielzahl von Durchtrittslöchern (42) umfasst, die in und entgegen der Fließrichtung (46) des Wassers relativ zu dem Strahlreglergehäuse (12) bewegbar angeordnet ist, wobei ein vorgespanntes elastisches Element (36) die Strahlscheibe (26) mit einer Dichtungsfläche (39) entgegen der Fließrichtung (46) unterhalb eines vorgegebenen maximalen Fließdrucks gegen eine gehäusefeste Dichtungsfläche (37) presst und die Durchtrittslöcher (42) in Größe und/oder Verteilung derart angeordnet sind, dass die Strahlscheibe (26) bei geschlossener Sanitärarmatur den Strahlregler (10) tropfdicht abschließt, bei einer Wasserentnahme das Wasser durch die Durchtrittslöcher (42) strömt und bei Erreichen des vorgegebenen maximalen Fließdrucks die Dichtungsfläche (39) der Strahlscheibe (26) entgegen der Vorspannung von der gehäusefesten Dichtungsfläche (37) abhebt, so dass das Wasser zwischen den Dichtungsflächen (37, 39) durchströmen kann.

2. Niederdruck-Sanitärarmatur nach Anspruch 1, **dadurch gekennzeichnet, dass** das elastische Element eine Druckfeder (36) ist, die mit einem Ende insbesondere im Zentrum der Strahlscheibe (26) angreift und mit dem anderen Ende an einem gehäusefesten Teil (31) anliegt.

3. Niederdruck-Sanitärarmatur nach Anspruch 2, **dadurch gekennzeichnet, dass** die Druckfeder (36) auf einem zum Strahlreglergehäuse (12) koaxialen Führungsbolzen (28) sitzt, der mit der Strahlscheibe (26) relativ zu dem Strahlreglergehäuse (12) bewegbar ist.

4. Niederdruck-Sanitärarmatur nach Anspruch 3, **dadurch gekennzeichnet, dass** der Strahlregler ein Anzeigemittel (44) aufweist, mit dem die Position des Führungsbolzens (28) von außen erkennbar ist.

5. Niederdruck-Sanitärarmatur nach einem der vorigen Ansprüche, **dadurch gekennzeichnet, dass** die gehäusefeste Dichtungsfläche (37) von einem Dichtungsring (38) mit gebildet ist, der an dem Strahlreglergehäuse (12) auf der dem Wasserauslauf zugewandten Stirnseite angeordnet ist.

6. Niederdruck-Sanitärarmatur nach einem der vorigen Ansprüche, **dadurch gekennzeichnet, dass** die Strahlscheibe (26) bei Überschreiten eines Fließdrucks von 0,5 bar abhebt.

7. Niederdruck-Sanitärarmatur nach einem der vorigen Ansprüche, **dadurch gekennzeichnet, dass** die Durchtrittslöcher (42) wabenartig angeordnet sind.

## Claims

1. Low-pressure sanitary fitting for use with a non-pressurized boiler, having
a) a water outlet; and
b) a jet regulator;
- a jet regulator housing (12);
- an installation (26, 36) which prevents the subsequent dripping of water in the case of a closed sanitary fitting;
**characterized in that**
the installation for preventing the subsequent dripping comprises a spray disc (26) which has a multiplicity of passage holes (42) and which relative to the jet regulator housing (12) is disposed movably in and counter to the flow direction (46) of the water, wherein a pretensioned elastic element (36) below a predefined maximum flow pressure urges the spray disc (26) by way of a sealing face (39), counter to the flow direction (46), against a sealing face (37) that is fixed to the housing, and the passage holes (42) in terms of size and/or distribution are disposed in such a manner that the spray disc (26) in the case of a closed sanitary fitting closes the jet regulator (10) in a drip-tight manner, the water in the case of a retrieval of water flows through the passage holes (42), and when reaching the predefined maximum flow pressure raises the sealing face (39) of the spray disc (26), counter to the pretensioning, from the sealing face (37) that is fixed to the housing such that the water can flow through between the sealing faces (37, 39).

2. Low-pressure sanitary fitting according to Claim 1, **characterized in that** the elastic element is a compression spring (36) which by way of one end engages in particular in the centre of the spray disc (26) and by way of the other end bears on a part (31) that is fixed to the housing.

3. Low-pressure sanitary fitting according to Claim 2, **characterized in that** the compression spring (36) sits on a guide pin (28) which is coaxial with the jet regulator housing (12) and which conjointly with the spray disc (26) is movable relative to the jet regulator housing (12).

4. Low-pressure sanitary fitting according to Claim 3, **characterized in that** the jet regulator has an indication means (44) by way of which the position of the guide pin (28) is able to be identified from the outside.

5. Low-pressure sanitary fitting according to one of the preceding claims, **characterized in that** the sealing face (37) that is fixed to the housing is conjointly formed by an annular seal (38) which is disposed on the jet regulator housing (12) on that end side that faces the water outlet.

6. Low-pressure sanitary fitting according to one of the preceding claims, **characterized in that** the spray disc (26) is raised when a flow pressure of 0.5 bar is exceeded.

7. Low-pressure sanitary fitting according to one of the preceding claims, **characterized in that** the passage holes (42) are disposed in a honeycomb manner.

## Revendications

1. Robinetterie sanitaire à basse pression destinée à être utilisée avec un accumulateur d'eau chaude sans pression avec
a) une sortie d'eau et
b) un régulateur de jet avec
- un boîtier de régulateur de jet (12);
- un dispositif (26, 36) qui empêche l'égouttage d'eau lorsque la robinetterie est fermée,
**caractérisée en ce que** le dispositif pour empêcher l'égouttage comprend un disque à jets (26) avec une multiplicité de trous de passage (42), qui est disposé de façon déplaçable dans la direction d'écoulement (46) de l'eau et inversement par rapport au boîtier de régulateur de jet (12), dans laquelle un élément élastique précontraint (36) presse le disque à jets (26) avec une face d'étanchéité (39) en opposition à la direction d'écoulement (46) en dessous d'une pression d'écoulement maximale prédéterminée contre une face d'étanchéité fixe du boîtier (37) et les trous de passage (42) sont disposés, en grandeur et/ou en distribution, de telle manière que le disque à jets (26) ferme de façon étanche aux gouttes le régulateur de jet (10) lorsque la robinetterie sanitaire est fermée, que l'eau s'écoule à travers les trous de passage (42) lors d'un prélèvement d'eau et que, lorsque la pression d'écoulement maximale est atteinte, la face d'étanchéité (39) du disque à jets (26) se soulève de la face d'étanchéité fixe du boîtier (37) contre la précontrainte, de telle manière que l'eau puisse s'écouler entre les faces d'étanchéité (37, 39).

2. Robinetterie sanitaire à basse pression selon la revendication 1, **caractérisée en ce que** l'élément élastique est un ressort de pression (36), qui agit avec une extrémité en particulier au centre du disque à jets (26) et s'applique avec l'autre extrémité sur une partie fixe du boîtier (31).

3. Robinetterie sanitaire à basse pression selon la revendication 2, **caractérisée en ce que** le ressort de pression (36) est appliqué sur un axe de guidage (28) coaxial au boîtier de régulateur de jet (12), qui est déplaçable avec le disque à jets (26) par rapport au boîtier de régulateur de jet (12).

4. Robinetterie sanitaire à basse pression selon la revendication 3, **caractérisée en ce que** le régulateur de jet présente un moyen d'affichage (44), avec lequel la position de l'axe de guidage (28) peut être détectée de l'extérieur.

5. Robinetterie sanitaire à basse pression selon l'une quelconque des revendications précédentes, **caractérisée en ce que** la face d'étanchéité fixe du boîtier (37) est également formée avec un anneau d'étanchéité (38), qui est disposé sur le boîtier de régulateur de jet (12) sur le côté frontal tourné vers la sortie d'eau.

6. Robinetterie sanitaire à basse pression selon l'une quelconque des revendications précédentes, **caractérisée en ce que** le disque à jets (26) se soulève lors du dépassement d'une pression d'écoulement de 0,5 bar.

7. Robinetterie sanitaire à basse pression selon l'une quelconque des revendications précédentes, **caractérisée en ce que** les trous de passage (42) sont disposés à la manière d'alvéoles.
